# EUROPEAN PATENT APPLICATION

(11) **EP 3 659 637 A1**
(43) Date of publication of application: **03.06.2020**
(21) Application number: 18850980.6
(22) Date of filing: 17.08.2018
(51) Int. Cl.: A61M 1/00

(54) **NOVEL PERFUSION AND SUCTION SYSTEM**

(30) Priority: 28.08.2017 CN 201710749931
(71) Applicant: Wang, Guohua, Chongqing 401122 (CN)
(72) Inventor: Wang, Guohua, Chongqing 401122 (CN)
(74) Representative: Raffay & Fleck
(86) International application number: PCT/CN2018/101166
(87) International publication number: WO 2019/042167

(57) **Abstract**

The disclosure discloses a novel irrigation and aspiration system. A pump (6) is disposed on an irrigation tube (2) for delivering irrigation fluid. The irrigation tube (2) is connected in series with a check valve (3) and then connected with a first port of a tee joint (4); a second port of the tee joint (4) is connected with a suction tube of a negative pressure device through an aspiration tube (5); an electronic choke device (7) is installed on the aspiration tube (5); a third port of the tee joint is connected with an irrigation and aspiration common tube (8) which is also connected with a terminal execution device. Both the electronic choke device (7) and the pump (6) are controlled by a host computer. The electronic choke device (7) is turned off when the pump (6) works; and the pump (6) is stopped when the electronic choke device (7) is turned on. The novel irrigation and aspiration system is in combination with a variety of medical terminal execution devices using simple tubes to form a gastric lavage machine, and can be matched with a gastrointestinal endoscope without water delivery function or surgical operating instruments. The system is simple in structure, convenient to operate, easy to dismount, clean and disinfect, and also integrates irrigation and aspiration functions and will not readily be blocked during aspiration, so that the workload of medical staffs and the running cost are greatly decreased.

## Description

### CROSS-REFERENCE TO RELATED APPLICATION

This application claims priority to Chinese Application No. 2017107499315 filed on August 28, 2017, entitled "Novel Irrigation and Aspiration System", the disclosure of which are hereby incorporated by reference in its entirety.

### FIELD OF TECHNOLOGY

The present disclosure relates to the technical field of medical instruments, and more particularly, to a novel irrigation and aspiration system.

### BACKGROUND

During modern medical diagnosis and treatment, it is often necessary to irrigate and lavage specific fluids for the diagnosis and treatment targets and to aspirate liquids or liquid-like substances that affect the diagnosis and treatment, so as to improve the accuracy of diagnosis, reduce missed diagnosis and misdiagnosis, improve the quality of treatment, reduce side effects caused by treatment, or decrease the harm by harmful substances to human body.

At present, clinically used medical devices with irrigation, lavage and aspiration functions are generally with two channels of irrigation and aspiration, and is controlled by a front-end internal blocking method in contact with liquid, especially negative pressure aspiration. This will lead to a dead space; and the irrigation and aspiration channels makes it difficult to clean and disinfect, which may cause the blockage of aspiration.

In the use of gastrointestinal endoscopy without water delivery function, by being connected with the plier nozzle, the vision field definition enhancer for gastrointestinal endoscope (Chinese patent ZL 201210128223.7) can provide convenient lavage already. However, it needs to configure additional gastrointestinal connectors and lavage tubes, and thus it has the disadvantage that the workload of the doctor is increased and the operation thereof is affected.

Medical endoscopes, especially gastrointestinal endoscopes have brought great difficulties to the cleaning and disinfection of endoscopes, and are prone to cross contamination due to their complicated internal tubes and front-end internal blocking control.

The existing gastric lavage machine also has the disadvantage of difficult cleaning and disinfection since the irrigation tubes are built in.

The existing irrigation and aspiration devices used in surgery also have the disadvantages of inconvenient switching between irrigation washing and aspiration, easy blockage of aspiration, requirement for special assistance since the operation handle is connected with two tubes for irrigation and aspiration respectively, difficulty in cleaning and disinfection of the irrigation and aspiration tube supplies, and high cost of disposable items.

### SUMMARY

In view of the deficiencies above of the prior art, the technical problem to be solved by the present disclosure is to provide a novel irrigation and aspiration system.

The technical scheme of the present disclosure is as follows: a novel irrigation and aspiration system, wherein a pump is arranged on an irrigation tube for delivering irrigation fluid, the irrigation tube is connected in series with a check valve and is then connected with a first port of a tee joint, a second port of the tee joint is connected with a suction tube of a negative pressure device through an aspiration tube, an electronic choke device is installed on the aspiration tube, a third port of the tee joint is connected with an irrigation and aspiration common tube, and the irrigation and aspiration common tube is connected with terminal execution devices; both the electronic choke device and the pump are controlled by a host computer, the electronic choke device is turned off when the pump works; the pump is stopped when the electronic choke device is turned on.

The electronic choke device is controlled by a first delayer provided in the host computer, and may be turned off in a delayed manner (purpose of the delayed stop: to completely aspirate the "dirty liquid" in the irrigation and aspiration common tube). The pump is controlled by an automatic controller provided in the host computer. When the electronic choke device is turned off in a delayed manner, the pump is automatically turned on to irrigate for a certain period of time (the purpose of automatic irrigation is to pre-fill the completely aspirated irrigation and aspiration common tube with an irrigation liquid), and the irrigation time is controlled by a second delayer provided in the host computer. The above scheme is controlled automatically by the host computer (the turn-off is delayed for a certain period of time after the artificial aspiration is stopped, and automatic irrigation is then performed for a certain period of time), so that the irrigation and aspiration can share the same tube as longer as possible, and it is possible that the fluid in the common tube is ready for use at any time. In this way, the complexity of the tube is reduced, clinic cleaning and disinfection are easily performed, it is convenient to operate, and the production costs are lowered. It can also ensure the thoroughness of aspiration and the lavage efficiency is effectively improved.

The host computer is provided with a first selection rotary knob by which the turn-off of the electronic choke device is delayed, and the first selection rotary knob has a plurality of tap positions; the host computer is further provided with a second rotary knob for controlling the automatic irrigation time after the turn-off of the electronic choke device is delayed, and the second selection rotary knob has a plurality of tap positions. The structure above may select the time at which the aspiration delay is stopped and the time for the automatic irrigation according to the actual situations such as the length and size of the irrigation and aspiration common tube, so as to meet different usage needs.

The tee joint has a "T" shape, the second and third ports of the tee joint are located on a straight line, and a check valve is installed at the first port of the tee joint. The structure above in combination with the electronic choke device may ensure the unobstructed property of an aspiration channel, and can effectively prevent an aspirate from entering the irrigation tube.

The present disclosure may not only irrigate and lavage, but also aspirate by combining a simple tube with a variety of medical terminal execution devices, and has the features as follows:
1. This system integrates irrigation and aspiration, and the tube connection is very simple. By selecting different terminal execution devices, it has multiple functions accordingly, which greatly saves the cost spent in adding equipment for the hospitals.
2. The front ends of the terminal execution devices share one channel which may be either long or short, and thus this system can meet different usage needs.
3. The system is automatically controlled by the host computer, which can make the irrigation fluid in the common channel ready for use at any time, and thus the lavage efficiency is greatly improved.
4. This system adopts a rear-mounted external blocking (non-contact) electronic control method, so that the tube has no dead space, is not easily blocked, is easy to be cleaned and disinfected, and is more convenient to clinically operate; in addition, the external tubes can be either reused after being cleaned and disinfected or disposable.

The terminal execution device is a gastrointestinal endoscope, and the suction port of the gastrointestinal endoscope is connected with the irrigation and aspiration common tube; a wireless or wired button, which replaces the original gastrointestinal endoscope suction button and is installed in the original location, is provided to control the turn-on and turn-off of the electronic choke device, the wireless or wired button seals this location of the gastrointestinal endoscope and keeps the suction channel unobstructed; and the startup and stop of the pump is controlled by a foot pedal. The scheme above, using the suction channel provided by the gastrointestinal endoscope, may both aspirate and irrigate and lavage, and is particularly suitable for the gastrointestinal endoscopes without water delivery function. Both irrigation, lavage and aspiration functions are realized in the original suction channel, which can prevent the suction channel from being blocked, and is particularly suitable for the diagnosis and treatment of emergency patients with insufficient preparation of the gastrointestinal tract, and does not change the operating habits of the medical staffs, for whom the scheme is very adaptable.

The terminal execution device is a gastric lavage tube, an electronic suction button that controls the turn-on and turn-off of the electronic choke device is installed on the host computer, and an electronic irrigation button that controls the startup and stop of the pump is also installed on the host computer. The structure above is easy to dismount, clean and disinfect, and overcomes the disadvantages that the existing gastric lavage machines are difficult to clean and disinfect due to the built-in irrigation and aspiration tube.

The terminal execution device is a surgical instrument, an electronic suction button that controls the turn-on and turn-off of the electronic choke device is installed on the surgical instrument, and an electronic irrigation button that controls the startup and stop of the pump is also installed on the surgical instrument. The structure above is used for irrigation and aspiration of minimally invasive surgery. When this system is connected to surgical instruments, the conversion of irrigation, lavage and aspiration is simple and convenient, and the surgeon may convert it by himself/herself without the assistance from others.

Beneficial effects: The present disclosure is in combination with a variety of medical terminal execution devices using simple tubes to form a gastric lavage machine, or is matched with a gastrointestinal endoscope without water delivery function or surgical operating instrument. The system is simple in structure, convenient to operate, easy to dismount, easy to clean and disinfect; and it also integrates irrigation and aspiration functions and will not readily be blocked during aspiration, so that the workload of medical staffs and the running cost are greatly decreased.

### BRIEF DESCRIPTION OF THE DRAWINGS

- Fig. 1: is a schematic structural diagram of Embodiment 1;
- Fig. 2: is a schematic structural diagram of Embodiment 2; and
- Fig. 3: is a schematic structural diagram of Embodiment 3.

### DETAILED DESCRIPTION

The present disclosure will be further described below with reference to the drawings and embodiments.

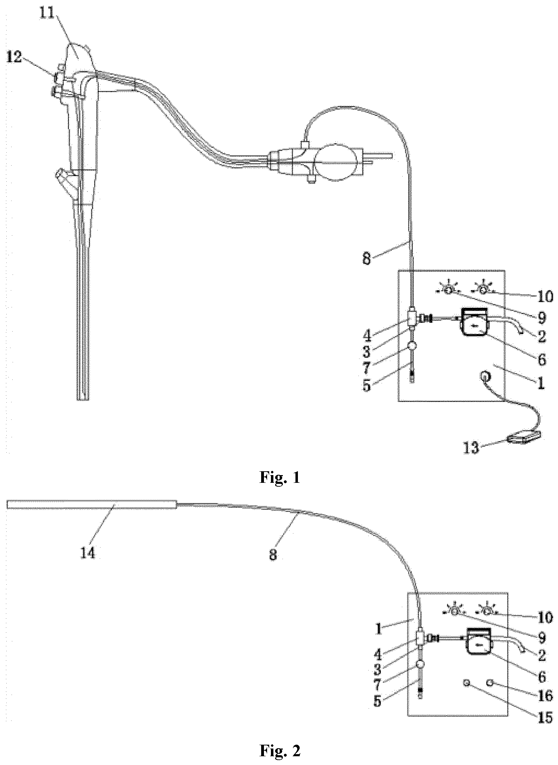

As shown in Figs. 1 to 3, the present disclosure comprises a host computer 1, an irrigation tube 2, a check valve 3, a tee joint 4, an aspiration tube 5, a negative pressure aspiration device, a pump 6, an electronic choke device 7, an irrigation and aspiration common tube 8, and a terminal execution device. In an embodiment, a pump 6 is disposed on the irrigation tube 2 for delivering the irrigation fluid, and the pump 6 is preferably a peristaltic pump. One end of the irrigation tube 2 is connected to an irrigation fluid container (not shown in the figures), and the other end of the irrigation tube 2 is connected in series with the check valve 3 and then connected with the first port of the tee joint 4. Preferably, the tee joint 4 has a "T" shape, and a check valve 3 is installed at the first port of the tee joint 4, and the second port and the third port of the tee joint 4 are located on a straight line. The second port of the tee joint 4 is connected to the suction tube of the negative pressure device through the aspiration tube 5. An electronic choke device 7 is installed on the aspiration tube 5. The electronic choke device 7 is preferably an electronic tube clip. The third port of the tee joint 4 is connected with an irrigation and aspiration common tube 8 which is also connected to a terminal execution device.

As shown in Figs. 1 to 3, the electronic choke device 7 and the pump 6 are both controlled by the host computer 1. The electronic choke device 7 is turned off when the pump 6 works; the pump 6 is stopped when the electronic choke device 7 is turned on. Preferably, the electronic choke device 7 is controlled by the first delayer provided in the host computer 1 and may be turned off in a delayed manner. The host computer 1 is provided with a first selection rotary knob 9 by which the turn-off of the electronic choke device 7 is delayed. The first selection rotary knob 9 has a plurality of tap positions. For instance: the first selection rotary knob has 4 tap positions, with the shortest time being 0 seconds, and the longest time being 3 seconds. The pump 6 is controlled by an automatic controller provided in the host computer 1. When the turn-off of the electronic choke device 7 is delayed, the pump 6 is automatically turned on to irrigate for a certain period, and the irrigation time is controlled by the second delayer provided in the host computer 1. The host computer 1 is provided with a second selection rotary knob 10 that controls the automatic irrigation time after the turn-off of the electronic choke device 7 is delayed. The second selection rotary knob 9 has multiple tap positions. For instance, the second selection rotary knob has 5 tap positions, with the shortest time being 0 seconds, and the longest time being 4 seconds.

According to different terminal execution devices, the present disclosure has various variations, specifically as follows:

### Embodiment 1

As shown in Fig. 1, this embodiment improves the function of the existing gastrointestinal endoscopes. The terminal execution device is a gastrointestinal endoscope 11 of which a suction port is connected to the irrigation and aspiration common tube 8. The startup and stop of the pump 6 is controlled by the foot pedal 13. A wireless or wired button 12, which replaces the original gastrointestinal endoscope suction button and is installed in the original location, is provided to control the turn-on and turn-off of the electronic choke device 7, and the wireless or wired button 12 seals this location of the gastrointestinal endoscope 11 and keeps the suction channel unobstructed.

The working principle of embodiment 1 is as follows:
The delayed turn-off time is set by the first selection rotary knob 9, and the pre-lavage time is set by the second selection rotary knob 10. The medical staff holds the handle of the gastrointestinal endoscope 11 and presses the wireless or wired button 12 to turn on the electronic choke device 7, and stop the pump 6, and thus the aspiration function is achieved under the action of the negative pressure device. When the aspiration is completed, the wireless or wired button 12 is released, and the electronic choke device 7 continues to be turned on for a certain period of time until the negative pressure device automatically stops working; at this time, the pump 6 automatically starts, so that the irrigation fluid passes through the irrigation tube 2 and the irrigation and aspiration common tube 8 sequentially, and then enters the irrigation and aspiration common channel of the gastrointestinal endoscope 11 to realize pre-irrigation, the pump 6 automatically stops after it works for a few seconds; in order to achieve continuous irrigation and lavage, the foot pedal 13 is pressed so that the pump 6 continue to work.

### Embodiment 2

As shown in Fig. 2, this embodiment relates to novel gastric lavage machine, and the terminal execution device is a gastric lavage tube 14. An electronic suction button 15 that controls the turn-on and turn-off of the electronic choke device 7 is installed on the host computer 1, and an electronic irrigation button 16 that controls the startup and stop of the pump 6 is also installed on the host computer 1. The working principle of irrigation and aspiration in this embodiment is the same as that in Embodiment 1, so details are not described herein.

### Embodiment 3

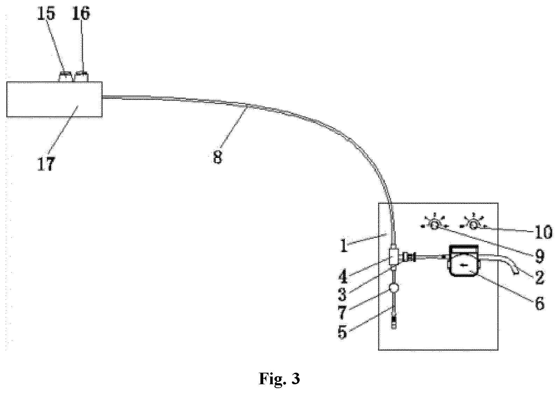

As shown in Fig. 3, this embodiment is used for irrigation and aspiration of minimally invasive surgery, and the terminal execution device is a surgical instrument 17. An electronic suction button 15 that controls the turn-on and turn-off of the electronic choke device 7 is installed on the surgical instrument 17, and an electronic irrigation button 16 that controls the startup and stop of the pump 6 is also installed on the surgical instrument 17. The working principle of irrigation and aspiration in this embodiment is the same as that in Embodiment 1, so details are not described herein.

## Claims

1. A novel irrigation and aspiration system, **characterized in that** a pump (6) is disposed on an irrigation tube (2) for delivering irrigation fluid, the irrigation tube (2) is connected in series with a check valve (3) and then connected with a first port of a tee joint (4); a second port of the tee joint (4) is connected with a suction tube of a negative pressure device through an aspiration tube (5); an electronic choke device (7) is disposed on the aspiration tube (5); a third port of the tee joint (4) is connected with an irrigation and aspiration common tube (8); and the irrigation and aspiration common tube (8) is connected with a terminal execution device; both the electronic choke device (7) and the pump (6) are controlled by a host computer (1), the electronic choke device (7) is turned off when the pump (6) works; and the pump (6) is stopped when the electronic choke device (7) is turned on.

2. The novel irrigation and aspiration system of claim 1, **characterized in that** the electronic choke device (7) is controlled by a first delayer provided in the host computer (1), and is turned off in a delayed manner; the pump (6) is controlled by an automatic controller provided in the host computer (1), when the turn-off of the electronic choke device (7) is delayed, the pump (6) is automatically turned on to irrigate for a certain period of time, and the irrigation time is controlled by a second delayer provided in the host computer (1).

3. The novel irrigation and aspiration system of claim 2, **characterized in that** the host computer (1) is provided with a first selection rotary knob (9) by which the turn-off of the electronic choke device (7) is delayed, and the first selection rotary knob (9) has a plurality of tap positions; the host computer (1) is further provided with a second rotary knob (10) for controlling the automatic irrigation time after the turn-off of the electronic choke device (7) is delayed, and the second selection rotary knob (10) has a plurality of tap positions.

4. The novel irrigation and aspiration system of claim 1 or 2 or 3, **characterized in that** the tee joint (4) has a "T" shape, the second port and the third port of the tee joint (4) are located on a straight line, and a check valve (3) is installed at the first port of the tee joint (4).

5. The novel irrigation and aspiration system of claim 4, **characterized in that** the terminal execution device is a gastrointestinal endoscope (11), and the suction port of the gastrointestinal endoscope (11) is connected with the irrigation and aspiration common tube (8); a wireless or wired button (12), which replaces the original gastrointestinal endoscope suction button and is installed in the original location, is provided to control the turn-on and turn-off of the electronic choke device (7), and the wireless or wired button (12) seals this location of the gastrointestinal endoscope (11) and keeps the suction channel unobstructed; the startup and stop of the pump (6) is controlled by a foot pedal (13).

6. The novel irrigation and aspiration system of claim 4, **characterized in that** the terminal execution device is a gastric lavage tube (14), an electronic suction button (15) that controls the turn-on and turn-off of the electronic choke device (7) is installed on the host computer (1), and an electronic irrigation button (16) that controls the startup and stop of the pump (6) is also installed on the host computer (1).

7. The novel irrigation and aspiration system of claim 4, **characterized in that** the terminal execution device is a surgical instrument (17), an electronic suction button (15) that controls the turn-on and turn-off of the electronic choke device (7) is installed on the surgical instrument (17), and an electronic irrigation button (16) that controls the startup and stop of the pump (6) is also installed on the surgical instrument (17).
